(19) Europäisches Patentamt
European Patent Office
Office européen des brevets

(11) Publication number: 0 392 663 A1

(12) # EUROPEAN PATENT APPLICATION

(21) Application number: 90302656.5

(51) Int. Cl.5: A61K 31/44

(22) Date of filing: 13.03.90

The title of the invention has been amended (Guidelines for Examination in the EPO, A-III, 7.3).

(30) Priority: 13.03.89 JP 60318/89

(43) Date of publication of application: 17.10.90 Bulletin 90/42

(84) Designated Contracting States: AT BE CH DE DK ES FR GB GR IT LI LU NL SE

(71) Applicant: ONO PHARMACEUTICAL CO., LTD.
1-5, Doshomachi 2-chome
Chuo-ku Osaka 541(JP)

(72) Inventor: Okegawa, Tadao, Minase Research Institute
Ono Phar. Co. Ltd., 3-1-1 Sakurai
Shimamoto-cho
Mishima-gun, Osaka(JP)
Inventor: Kawamura, Masanori, Minase Research Institute
Ono Phar. Co. Ltd., 3-1-1 Sakurai
Shimamoto-cho
Mishima-gun, Osaka(JP)

(74) Representative: Bentham, Stephen et al
J.A. Kemp & Co. 14 South Square Gray's Inn
London WC1R 5EU(GB)

(54) **Carboline derivative as a 5-HT3 receptor antagonist.**

(57) The present invention provides γ-carbolines of the formula:

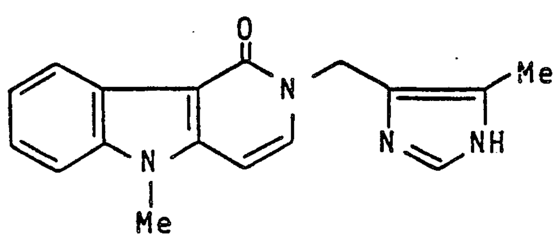

(I)

or non-toxic acid additional salts thereof and/or hydrates thereof, for use as 5-$HT_3$ receptor antagonists. The present invention also provides pharmaceutical compositions comprising compounds of the formula I.

EP 0 392 663 A1

## 5-$HT_3$ RECEPTOR ANTAGONIST

The present invention relates to the use of a -carboline derivative of formula I:

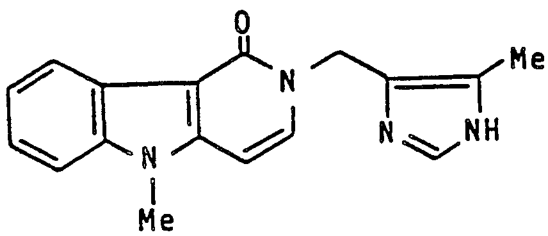

(I)

and non-toxic acid addition salts thereof and hydrates thereof as a 5-$HT_3$ receptor antagonist. It will be clear to those skilled in the art that the imidazole moiety of the compound of formula I may exist in tautomeric forms.

5-HT (hydroxytryptamine, serotonin) is a neurotransmitter in the living body. Three types of receptor which are related to 5-HT are known, and they are called 5-$HT_1$, 5-$HT_2$ and 5-$HT_3$ type receptors.

5-$HT_3$ receptors are widely distributed in the brain, heart, digestive canal, and 5-HT has an activity to these receptors as a mediator.

When 5-HT acts on 5-$HT_3$ receptors at peripheral nerves symptoms including pain and bradycardia are induced; whereas when 5-HT acts on 5-$HT_3$ receptors of the central nerve, sensory responses such as emotions, appetite, and memory are triggered. Furthermore, 5-HT acts on 5-$HT_3$ receptor at the CTZ (chemoreceptor trigger zone) in the brain, which induces nausea and vomiting.

It is therefore believed that 5-$HT_3$ receptor antagonists are useful for the prevention and treatment of diseases and conditions related to the central nerves such as schizophrenia, corpulence, mania, anxiety, gastroenteric functional disorders such as peptic ulcer, peptic esophagitis and migraine, vertigo, nausea, vomiting (especially, vomiting induced by administration of anti-cancer agents such as cisplatin).

It is known that administration of the anti-emetic drug metoclopramide, (N-[2-diethylamino)ethyl]-2-methoxy-4-amino-5-chlorobenzamide) which causes side-effects, only suppress vomiting induced by anti-cancer agents, and any other anti-emetic agents are not effective 5-$HT_3$ receptor antagonists.

Certain $\gamma$-carboline and carbazole derivatives are known to have activity as 5-$HT_3$ receptor antagonists. For example, EP-A-306323 discloses compounds of the formula (A):

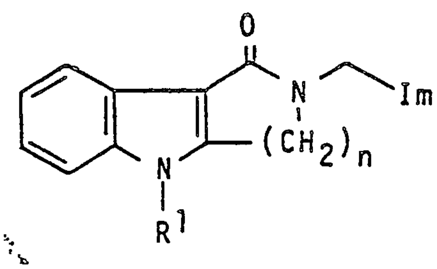

(A)

as 5-$HT_3$ receptor antagonists, wherein Im represents an imidazoyl group of the formula:

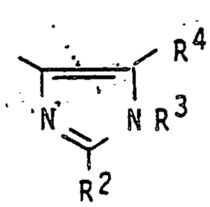

or

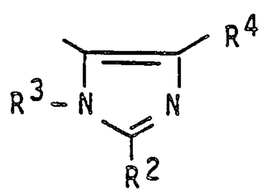

and $R^1$ represents a hydrogen atom or a group selected from $C_{1-6}$alkyl, phenyl, phenyl$C_{1-3}$alkyl, phenylmethoxymethyl, phenoxyethyl, phenoxymethyl, -$CO_2R^5$, -$COR^5$, -$CONR^5R^6$ (wherein $R^5$ and $R^6$, which may be the same or different, each represent a hydrogen atom, a $C_{1-6}$alkyl or $C_{3-7}$cycloalkyl group

or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$):
one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;
n represents 2 or 3;
and physiologically acceptable salts and solvates thereof.

Compounds of the formula (A) include the compound of the formula (a):

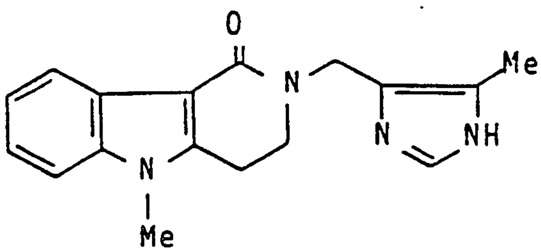

(a)

which is obtained by reduction of a compound of the formula (I). However, the pharmacological activity of the compound of the formula (I) is not disclosed.

Indeed, it is surprising that the compound of the formula (I) has pharmacological activity, i.e. 5-$HT_3$ receptor antagonist activity, since the specification only relates to the compound of the formula (I) as an intermediate for synthesis of the compound of the formula (a).

Because each step of a reaction scheme involves additional expense and, as a rule, a reduction in yield, those of skill in the art would be taught by EP-A-306323 that the reduction of the compound of formula I at the 3,4 double bond to a compound of formula (a) was a necessary step in order to obtain a compound with useful 5-$HT_3$ receptor-antagonist activity.

Furthermore, the compounds of the formula (A) are related structurally to carbazoles, which are known as 5-$HT_3$ receptor antagonists. All of the carbazole compounds known as 5-$HT_3$ antagonists have a structure which, like compounds of the formula (A) and unlike the compound of the formula I is 3,4-saturated (1,2 saturated in carbazoles).

Such carbazoles include those disclosed in the following references. (Unrelated definitions are omitted.)

- Great Britain Patent No. 2153821; formula:

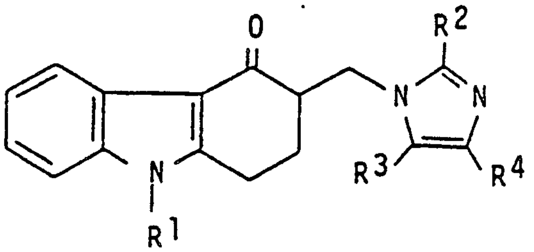

(B)

- European Patent Publication No. 191562; formula:

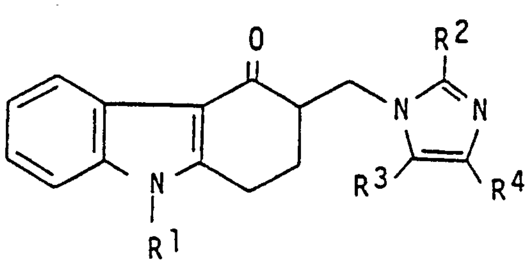

(C)

- European Patent Publication No. 219193; formula:

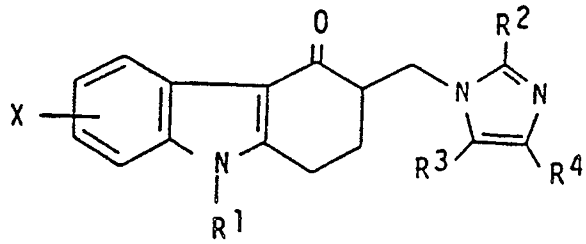

(D)

- European Patent Publication No. 210840; formula:

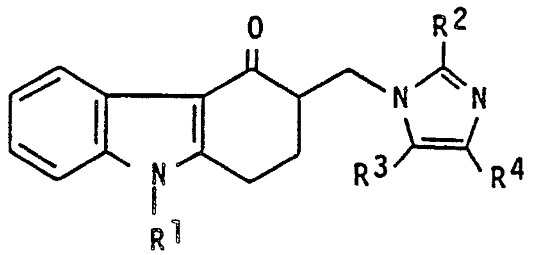

(E)

- Great Britain Patent Publication No. 2192885; formula:

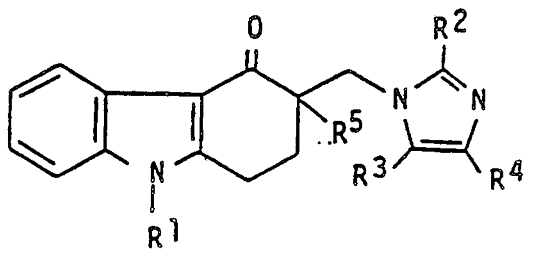

(F)

- Great Britain Patent Publication No. 2202530; formula:

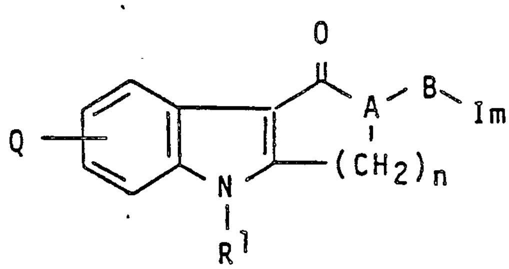

(G)

wherein n is 1, 2 or 3. A-B is $CH\text{-}CH_2$ or $C=CH$.

- European Patent Publication No. 297651; formula:

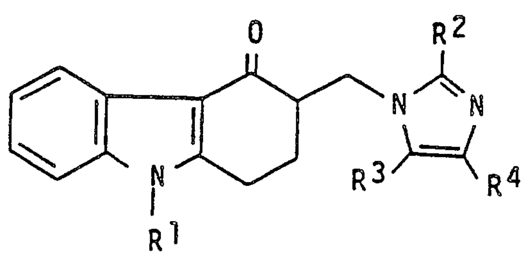

(H)

• European Patent Publication No. 338650; formula:

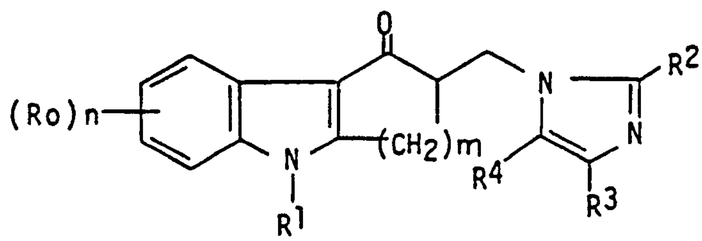

(J)

wherein m is 3-8.

Thus the trend in the art established by compounds of formula (B) to (J) which is followed by EP-A-306323 teaches those skilled in the art that compounds which are saturated at the 1st and 2nd positions of carbazoles and at the 3rd and 4th positions of γ-carbolines are effective as $5\text{-}HT_3$ receptor antagonists.

In direct contrast to this established prejudice, and contrary to expectations, we have found that 3,4-non-saturated (double-bonded)-γ-carboline possesses potent $5\text{-}HT_3$ receptor antagonistic activity.

In addition, the compounds of the present invention not only possess potent $5\text{-}HT_3$ receptor antagonist activity but also possess low toxicity. Therefore, the compound of the present invention is suitable for effective and safe for pharmaceutical use.

The present invention accordingly provides a method for the treatment or prophylaxis of diseases induced by the action of 5-hydroxy-tryptamine on 5-hydroxytryptamine 3-receptors in a mammalian host, which comprises administering to a host suffering from, or subject to the above diseases a derivative of γ-carboline of the formula:

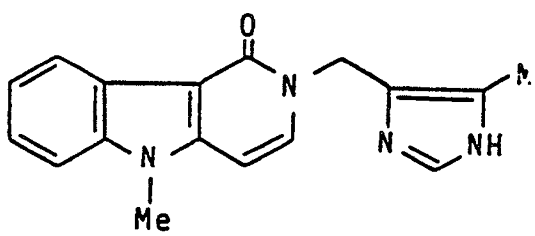

(I)

or acid addition salts thereof and/or hydrates thereof.

The present invention also provides a γ-carboline of the formula I:

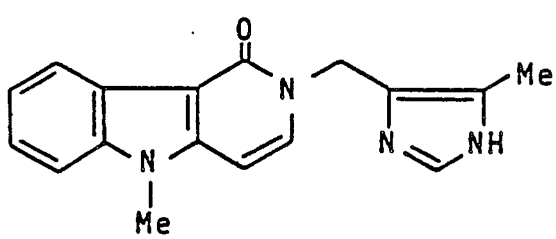

(I)

or acid addition salts thereof and/or hydrate thereof for use in a method of treatment of prophylaxis of diseases or conditions induced by the action of 5-hydroxytryptamine on 5-hydroxytryptamine-3-receptors in a mammalian, including man.

The invention further provides a pharmaceutical composition comprising, as active ingredient, a γ-carboline derivative of formula I or a pharmaceutically acceptable acid addition salt thereof and/or hydrate thereof in association with a pharmaceutically acceptable carrier or coating.

It will be clear to those skilled in the art that a composition consisting of the active ingredient alone, or consisting of the active ingredient dissolved in absolute ethanol alone, (which are disclosed in EP-A-306323) are excluded from the above definition of pharmaceutical compositions.

The compounds of the present invention of the formula (I) may be prepared by N-acylation of a compound of formula:

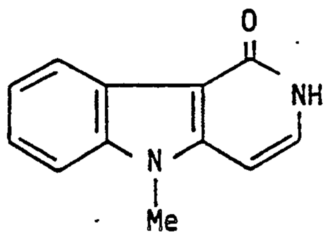

(II)

(wherein all of the symbols have the same meaning as defined hereinbefore) with a compound of formula:

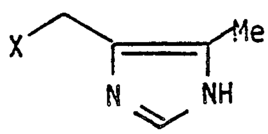

(III)

(wherein X is halogen atom, and the other symbols are as hereinbefore defined).

N-alkylation is a known reaction and may be carried out, for example, in a polar solvent (ethyl either, THF, acetonitrile, DMF, HMPA, etc.), in the presence of a base (e.g. sodium hydride).

The compound of the formula (II) may be prepared according to the reaction scheme (A) described below.

All these reactions in the following reaction schemes are known and the symbols provided therein are as hereinbefore defined except for $R^{50}$ which represents $C_{1-4}$alkyl.

Reaction Scheme (A)

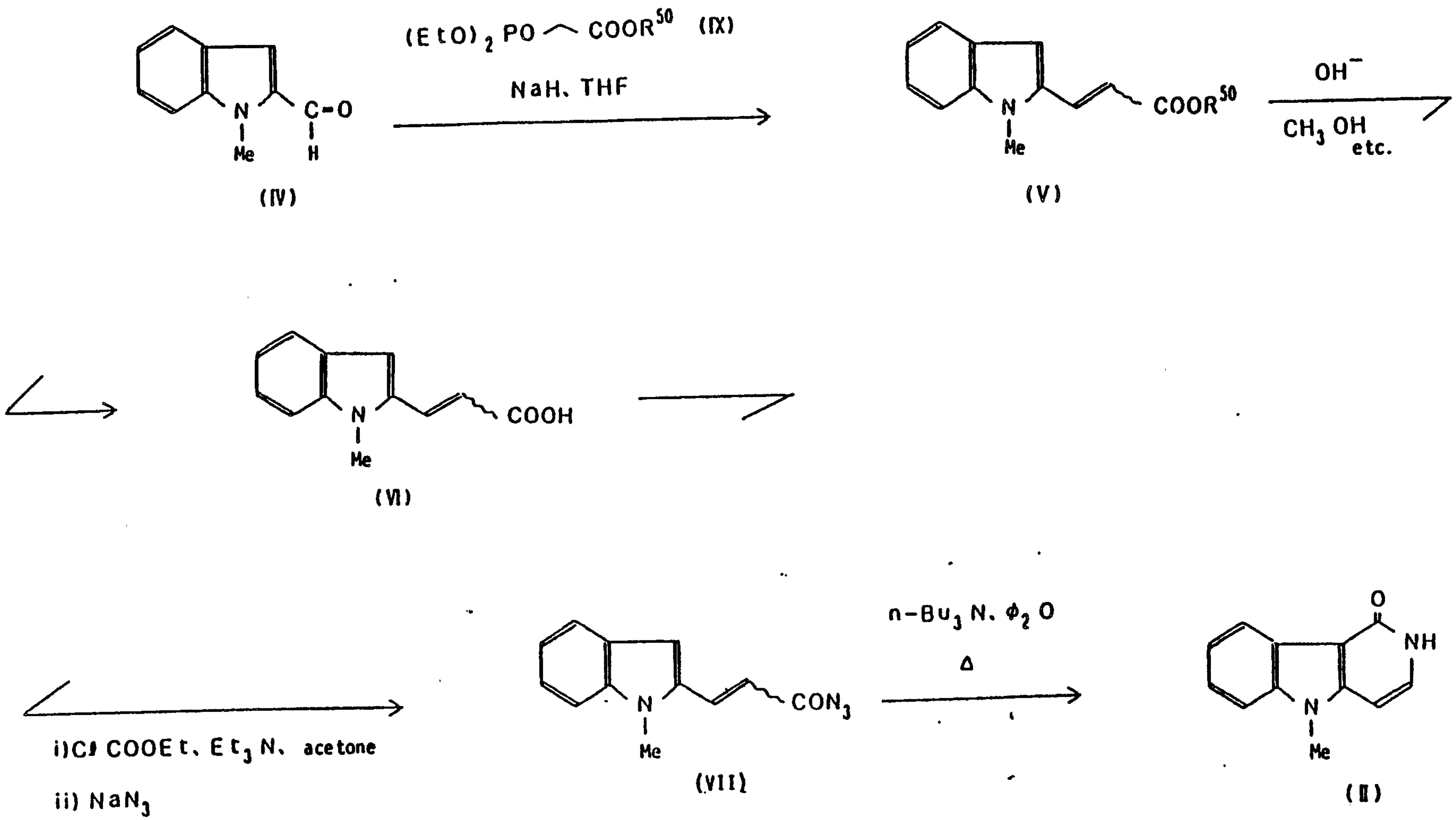


(IV) (V) (VI) (VII) (II)

Starting materials and reagents for use in the present invention are known per se or may be prepared by known methods.

For example, the compound of the formula (III) wherein X is chlorine is available in the market.

For example, the compound of the formula (IV) is described in Chem.Abst., 59, 3899b (1963).

The compounds of the formula (I) may be converted into the corresponding acid additional salts. Non-toxic and water-soluble salts are preferable. Suitable salts, for example, are followings: salts of inorganic acids e.g. hydrochloride, hydrobromide, sulphate, phosphate, nitrate etc. Salts of organic acids e.g. acetate, lactate, tartarate, fumarate, maleate, oxalate, citrate, benzoate, methanesulphonate, ethanesulphonate, benzenesulphonate, toluenesulphonate, isethioate, glucuronate, gluconate etc. Hydrochloride is preferable.

The compounds of the formula (I) or salts thereof may be converted into a hydrate by conventional means.

The compounds of the present invention of the formula (I) possess an antagonistic activity against 5-$HT_3$ receptor as described above. For example, in a standard laboratory test, 2-(5-methylimidazol-4-ylmethyl)-5-methyl-1, 2,-dihydro-γ-carbolin-1-one hydrochloride shows an antagonistic activity against 5-$HT_3$ receptor in vivo in rats, with an $ID_{50}$ i.v. of 0.3 mg/kg and an $ID_{50}$ i.d. of 7.7 mg/kg.

Male Wistar rats were urethane-anesthetized and fixed. Cannulas were inserted to carotid artery and thigh vein as used for the recording of blood pressure end heart beat, and for the administration of compounds, respectively.

Several amounts of compound of the present invention were administered to the vein or duodenum. 5-HT was administered rapidly to the vein, 2 mins after. The suppression effect of the compounds was confirmed by the measurement of reflex bradycardia generated. (Nature 316, 11 (1985).)

It was confirmed that the toxicity of the compounds of the present invention were very low. Therefore, the compounds of the present invention may be considered to be sufficiently safe and suitable for pharmaceutical use.

For example, the values of acute toxicity ($LD_{50}$) of 2-(5-methylimidazol-4-ylmethyl)-5-methyl-1,2-dihydro-γ--carbolin-1-one hydrochloride was 290 mg/kg animal body weight by oral administration and 48 mg/kg animal body weight by intravenous administration in mice, respectively.

It is believed that the blocking of the activity of 5-$HT_3$ receptor is useful for the prevention and the treatment of diseases and condition associated with the central nerves such as schizophrenia, corpulence, mania, axiety, gastroenteric functional disorders such as peptic ulcer, peptic esophagitis and migraine, vertigo, nausea, vomiting (especially, vomiting induced by administration of anti-cancer agent such as cisplatin) in mammals including human beings.

The compound of the present invention possesses an antagonistic activity against 5-$HT_3$ receptor in vivo as shown in the experimental results above, so it is expected to be useful for the treatment or prophylaxis of the conditions described above.

For the purpose described above, the compounds of the present invention of the formula (I), pharmaceutically acceptable acid addition salts thereof and/or hydrates thereof may be administered systemically or partially, for example by oral or parenteral administration.

The doses to be administered will be determined depending upon age, body weight, symptoms, the desired therapeutic effect, the route of administration, and the duration of the treatment, and will be ultimately determined clinically by the skilled physician. In the human adult, the doses per person per dose are generally from 50 μg to 100 mg preferably from 1 mg to 20 mg, by oral administration, up to several times per day, and from 5 μg to 10 mg preferably 500 μg to 10 mg, by parenteral administration up to several times per day, or continuous adminstration between 1 and 24 hrs per day intravenously.

As mentioned above, the doses to be used depend upon various conditions. Therefore, there are cases in which doses lower than or greater than the ranges specified above may be used.

Compounds of the present invention may be administered as solid compositions, liquid compositions or other compositions for oral administration, and as injections, liniments or suppositories etc. for parenteral administration.

Solid compositions for oral administration include tablets, pills, capsules, dispersible powders, granules. Capsules include soft capsules and hard capsules.

Liquid compositions for oral administration include pharmaceutically acceptable-emulsions, solutions, suspensions, syrups and elixirs. Such compositions may be comprised in inert diluent(s) commonly used, for example water or ethanol.

Injections for parenteral administration include sterile aqueous or non-aqueous solutions, suspensions and emulsions.

Other compositions for parenteral administration include liquids for external use e.g. external solution,

endermic liniments, ointments, suppositories for rectal administration and pessaries.

The following examples and reference examples illustrate the present invention without limitation.

The solvents in the parentheses show the developing or eluting solvents and the ratio of the solvents used are by volume in chromatographic separations.

Unless otherwise specified, "IR" were measured by KBr tablet method.

The compound of the present invention are named as derivatives of γ-carboline numbered in the following manner:

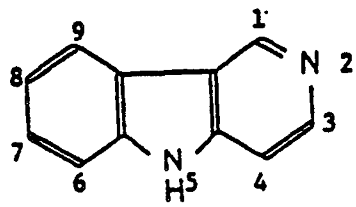

Reference example 1

Preparation of (2E)-3-(1-methylindol-2-yl)acrylic acid ethyl ester

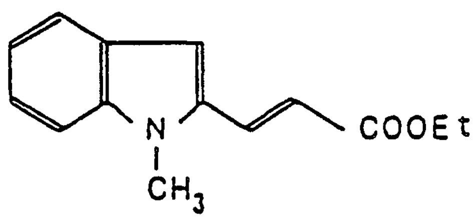

In an atmosphere of argon, sodium hydride (998 mg; 62% contents) was suspended in THF (40 ml), at 0°C. Diethyl ethoxycarbonylmethyl phosphorate (5.9 ml) was added dropwise to the suspension at the same conditions. The mixture was stirred for 1 hr, at the same conditions. After reaction, a solution of 1-methylindole-2-carbaldehyde (1.8 g) in THF (10 ml) was added dropwise to the solution, and the mixture was stirred for 30 mins. The reaction mixture was poured into an aqueous saturated solution of ammonium chloride. The mixture was extracted with ethyl acetate. The oily layer was washed, dried and evaporated. The residue was purified by column chromatography on silica gel (hexane : EtOAc = 10 : 1) to give the title compound (2.5 g) having the following physical data:
TLC : Rf 0.76 (hexane : EtOAc = 2 : 1)

Reference example 2

Preparation of (2E)-3-(1-methylindol-2-yl)acrylic acid

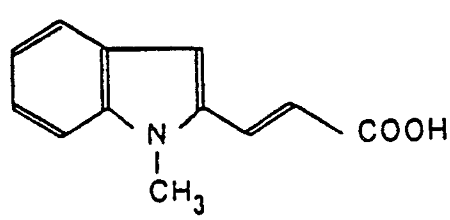

The compound prepared in reference example 1 (959 mg) was dissolved in a mixture of methanol and dioxan (10 ml-10 ml). 1N sodium hydroxide (5 ml) was added to the solution, and the solution was stirred for 16 hrs at room temperature. 1N hydrochloride was added to the solution, and the mixture was extracted with a mixture of EtOAc and chloroform. The oily layer was washed, dried and evaporated to give the title compound (880 mg) having the following physical data:
TLC : Rf 0.52 (chloroform : methanol = 5 : 1)

Reference example 3

Preparation of (2E)-3-(1-methylindol-2-yl)acryloylazide

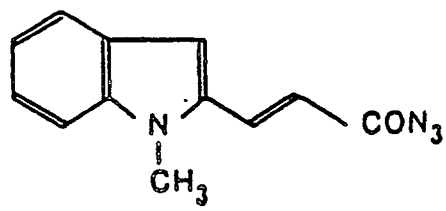

The compound prepared in reference example 2 (2.2 g) and triethylamine were dissolved in acetone. Ethyl chloroformate (1.25 ml) was added dropwise to the solution over a period of 5 mins at 0° C. The solution was stirred for 1.5 hrs at the same temperature. A solution of sodium azide (1.06 g) in water (3 ml) was dropped to the solution. The solution was stirred for 1 hr at 0° C. The reaction solution was evaporated keeping the temperature below 10° C. The residue was poured into ice-water. The mixture was extracted with methylene chloride. The oily layer was dried and filtrated. The filtrated was evaporated keeping the temperature below 20° C to give the title compound.

Reference example 4

Preparation of 5-methyl-1,2-dihydro-γ-carbolin-1-one

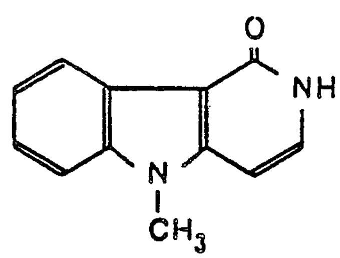

The compound prepared in reference example 3 was dissolved in diphenyl ether with heating. The solution was dropped to a mixed solution of tri-n-butylamine and diphenyl ether (2.78 ml - 10 ml) heating to 230° C. The solution was cooled to room temperature. Petroleum ether (40 ml) was added to the solution. Solids deposited were gathered by filtration, washed with petroleum ether, dried to give the title compound (1.71 g) having the following physical data:
TLC : Rf 0.48 (chloroform : methanol = 5 : 1);
IR : γ 2940, 2820, 1635, 1545, 1495, 1465, 1445, 1420, 1365, 1335, 1275, 1160, 1105, 1015, 991, 900 $cm^{-1}$

Reference example 5

Preparation of 2-(5-methylimidazol-4-ylmethyl)-5-methyl-1,2-dihydro-γ-carbolin-1-one hydrochloride

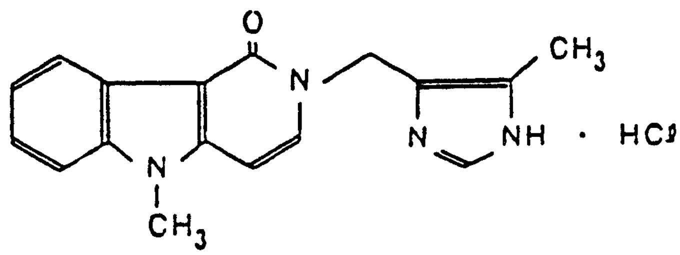

Sodium hydride (1.32 g; 62% contents) was added to a suspension of the compound prepared in reference example 4 (1.71 g) in DMF (20 ml), over a period of 4 mins. The reaction mixture was stirred for 20 mins at room temperature. A suspension of 4-chloro-5-methylimidazole hydrochloride (2.88 g) in DMF was added to the reaction mixture over a period of 5 mins. The mixture was stirred for 1 hr at room temperature. The reaction mixture was poured into ice-water.

The mixture was extracted with chloroform. The oily layer was dried and filtered. The filtrate was evaporated. The residue was purified by column chromatography (chloroform : methanol = 20 : 1). Powder obtained was dissolved in methanol.

4N hydrochloride-dioxan (3 ml) was added to the solution, and the solution was evaporated to dryness. The residue was washed with a mixture of methanol and ether, and ether, respectively, to give the title compound (980 mg) having the following physical data:
TLC : Rf 0.38 (chloroform : methanol = 8 : 1);
IR : γ 3410, 1645, 1570, 1470, 1370, 1240, 1185, 750 cm-1.

Example 1

The following components were admixed in conventional method and punched out to obtain 100 tablets each containing 20 mg of active ingredient.

| | |
|---|---|
| • 2-(5-methylimidazol-4-ylmethyl)-5-methyl-1,2-dihydro-γ-carbolin-1-one hydrochloride | 1.0 g |
| • Calcium cellulose glycolate (Carboxymethylcellulose calcium; disintegrating agent) | 0.2 g |
| • Magnesium stearate (lubricating agent) | 0.1 g |
| • Lactose | 8.7 g |

Example 2

The following components were admixed in conventional manner. The solution was sterilized in conventional manner, placed in 5 ml ampoules and freezed-dried to obtain 100 ampoules each containing 20 mg of the active ingredient.

| | |
|---|---|
| • 2-(5-methylimidazol-4-ylmethyl)-5-methyl-1,2-dihydro-γ-carbolin-1-one hydrochloride | 0.2 g |
| • lactose | 2 g |
| • distilled water | 100 ml |

**Claims**

1. A γ-carboline of the formula I

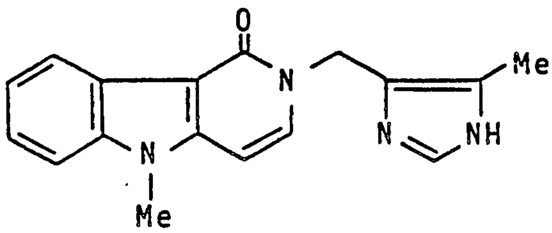

I

or pharmaceutically acceptable acid addition salt and/or hydrate thereof for use in a method of treatment or prophylaxis of diseases or conditions induced by the action of 5-hydroxytryptamine on 5-hydroxytryptamine 3-receptors in a mammal, including man.

2. A pharmaceutical composition comprising, as active ingredient, a γ-carboline of formula I

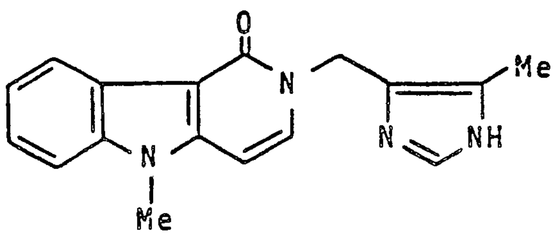

I

or pharmaceutically acceptable acid addition salt and/or hydrate thereof in association with a pharmaceutically acceptable carrier or coating.

3. A composition according to claim 2 in tablet form comprising the active ingredient and a solid carrier.

4. A composition according to claim 3 comprising 1-20 mg of the active ingredient.

5. A composition according to claim 2 comprising the active ingredient in a sterile aqueous solution.

6. A composition according to claim 5 comprising 0.5 to 10 mg of the active ingredient.

European Patent Office

# EUROPEAN SEARCH REPORT

Application Number

EP 90 30 2656

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 306 323 (GLAXO)<br>* Page 14, intermediate 18 *<br>--- | 1-6 | A 61 K 31/44 |
| Y,D | GB-A-2 153 821 (GLAXO)<br>* Entire document *<br>--- | 1-6 | |
| X,P | EP-A-0 356 098 (GLAXO)<br>* Entire document *<br>----- | 1-6 | |

TECHNICAL FIELDS SEARCHED (Int. Cl.5)

A 61 K 31/00

The present search report has been drawn up for all claims

| Place of search | Date of completion of the search | Examiner |
|---|---|---|
| THE HAGUE | 19-07-1990 | THEUNS H.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)